(19) **Europäisches Patentamt**
**European Patent Office**
**Office européen des brevets**

(11) **EP 1 930 419 B1**

(12) **EUROPEAN PATENT SPECIFICATION**

(45) Date of publication and mention
of the grant of the patent:
**03.11.2010 Bulletin 2010/44**

(21) Application number: **06810966.9**

(22) Date of filing: **25.09.2006**

(51) Int Cl.:
***C12N 9/00*** (2006.01)

(86) International application number:
**PCT/JP2006/319611**

(87) International publication number:
**WO 2007/034999 (29.03.2007 Gazette 2007/13)**

(54) **METHOD FOR PRODUCTION OF ENZYME**

ENZYMHERSTELLUNGSVERFAHREN

METHODE POUR PRODUIRE UNE ENZYME

(84) Designated Contracting States:
**DE DK**

(30) Priority: **26.09.2005 JP 2005278435**

(43) Date of publication of application:
**11.06.2008 Bulletin 2008/24**

(73) Proprietor: **Kao Corporation**
**Chuo-ku**
**Tokyo 103-8210 (JP)**

(72) Inventors:
• **SATO, Hitoshi**
**Kamisu-shi**
**Ibaraki 314-0103 (JP)**
• **SHOGA, Yutaka**
**Wakayama-shi**
**Wakayama 640-8580 (JP)**
• **KOYAMA, Shingo**
**Kamisu-shi**
**Ibaraki 314-0103 (JP)**
• **SHIRAKURA, Iyori**
**Kamisu-shi**
**Ibaraki 314-0103 (JP)**

(74) Representative: **HOFFMANN EITLE**
**Patent- und Rechtsanwälte**
**Arabellastrasse 4**
**81925 München (DE)**

(56) References cited:
**JP-A- 11 215 980**     **JP-A- 11 318 482**
**JP-A- 2003 514 563**     **US-A- 5 552 316**
**US-A1- 2004 110 264**

• **CORTEZ E V ET AL: "Liquid-liquid extraction of xylitol dehydrogenase from Candida guilliermondii homogenate by reversed micelles" JOURNAL OF CHROMATOGRAPHY B: BIOMEDICAL SCIENCES & APPLICATIONS, ELSEVIER, AMSTERDAM, NL, vol. 807, no. 1, 25 July 2004 (2004-07-25), pages 55-60, XP004512103 ISSN: 1570-0232**
• **REIS VAN R ET AL: "Membrane separations in biotechnology" CURRENT OPINION IN BIOTECHNOLOGY, LONDON, GB, vol. 12, no. 2, 1 April 2001 (2001-04-01), pages 208-211, XP002317550 ISSN: 0958-1669**

Remarks:
The file contains technical information submitted after the application was filed and not included in this specification

**Description**

Field of the invention

The present invention relates to a process for producing an enzyme, and in particular to a process for efficiently recovering an enzyme from an enzyme-containing solution.

Background of the invention

A method of recovering an objective fermentation product such as an enzyme by filtering a fermentation liquor and removing insolubles such as fine particles, cultured cells and spores in the fermentation liquor is known. However, a filtration membrane may, during a filtration operation, be clogged with particles similar in size to pores of the separation membrane, and insolubles are further accumulated on the membrane, to cause a problem of a reduction in filtration efficiency. Against such physical clogging of a separation membrane and accumulation of insolubles on a membrane, an operation that involves filtering a fermentation liquor and simultaneously flowing a sweeping stream on a membrane is conventionally carried out to avoid clogging and accumulation of insolubles, but in the case of a fermentation liquor containing a high density of microorganisms, the fermentation liquor is so viscous that pressure loss during flow of a sweeping stream is made great and filtration pressure is increased to consolidate insolubles on the membrane to adversely cause a problem of reduction in filtration efficiency.

To solve such problems, in JP-A 11-318482, a cationic surfactant is added to a fermentation culture containing a high density of microorganisms prior to microfiltration, to reduce the viscosity of the fermentation liquor, thereby suppressing pressure loss in the flow of a sweeping stream and increase in filtration pressure, thus improving the degree of concentration, whereby the recovery of a fermentation product is improved. JP-A 4-354585 has proposed addition of an aggregating agent (cationic high-molecular substance) to a fermentation culture in treating the fermentation liquor. However, the molecular weight of such high-molecular aggregating agent is several hundred thousand, and during repeated use thereof, a membrane is adversely clogged with the cationic polymer to cause a problem that the recovery of the membrane by washing may be made infeasible.

Summary of the invention

The present invention relates to a process for producing an enzyme, which includes recovering an enzyme by adding a cationic surfactant in an amount of 0.01 to 1% (w/v) to an enzyme-containing solution, having a cell density of 1% (v/v) or less and an enzyme concentration of 1% (w/v) or more in terms of the amount of proteins, and microfiltering the enzyme-containing solution and a process for producing an enzyme, which includes separating microbial cells from a fermentation culture having a cell density of more than 1% (v/v), purifying and concentrating the resulting enzyme-containing solution by ultrafiltration, adding a cationic surfactant in an amount of 0.01 to 1% (w/v) to the resulting enzyme-containing solution having a cell density of 1% (v/v) or less and an enzyme concentration of 1% (w/v) or more in terms of the amount of proteins and microfiltering the mixture.

Detailed description of the invention

In fermentative production of an enzyme such as protease, there is often used a method wherein a microorganism is first separated from a fermentation culture by microfiltration or the like, and then an enzyme-containing solution obtained by separating the microorganism is purified and concentrated by ultrafiltration.

By this method, a high conc. enzyme-containing solution having a cell density of 1% (v/v) or less and an enzyme concentration of 1% (w/v) or more can be obtained, but there is an increasing demand for further purification and separation of this high conc. enzyme-containing solution, from the viewpoint of complete prevention of an enzyme-producing microorganism, removal of saprophytic bacteria generated in a purification process, complete removal of insolubles generated by highly concentrating the enzyme solution and complete removal of a small amount of remaining impurities.

In purification and separation of this high conc. enzyme-containing solution, it is conceivable to employ a microfiltration membrane, but such high conc. enzyme-containing solution, though being a fermentation solution not problematic in viscosity because of its low cell density, was revealed to have a problem of inferior enzyme permeability and permeation flux in microfiltration and very poor production efficiency because of its high protein concentration.

Generally, the purification of a high-protein solution by microfiltration has problems such as a reduction in a permeation flux in membrane filtration and lower permeability of a protein solution, due to contaminating DNA etc. on the order of ppb even if the amount of impurities such as contaminating viruses to be removed is low. As a means of solving such problems, JP-A 2004-89155 proposes a method wherein for filtration of a protein solution through a membrane, the protein solution is treated with a deoxyribonuclease, that is Dnase, before or during filtration of the protein solution through the membrane. In a high conc. protease fermentation liquor, however, the Dnase itself is also a protein and is thus liable to decomposition with proteases, thus failing to exhibit its expected effect.

As described above, it was revealed that in purification by microfiltration, that is, in final purification and separation of a solution containing an enzyme at high concentration, there are problems such as very difficult realization of sufficient enzyme permeability and permeation flux and very low production efficiency.

The present inventors examined a method of realizing sufficiently high enzyme permeability and permeation flux in the final purification and separation of a highly concentrated enzyme-containing solution by using a microfiltration membrane, and as a result, they completed the present invention described above.

In the present invention, an enzyme-containing solution having a cell density of 1% (v/v) or less and an enzyme concentration of 1% (w/v) or more in terms of the amount of a protein is subjected to microfiltration to recover the enzyme. The cell density is more preferably 0.5% (v/v) or less, even more preferably 0 to 0.3% (v/v), from the viewpoint of reduction in the burden on microfiltration. The enzyme concentration is more preferably 2% (w/v) or more, even more preferably 2 to 10% (w/v), from the viewpoint of the solubility of the enzyme and the efficiency of filtration by the amount of a treated solution and the rate of filtration. The cell density is defined in terms of the ratio of the volume of microbial cells to the volume of the enzyme-containing solution, after centrifugation under the conditions of a centrifugal effect of 12000 G and a settling time of 5 min.

The enzyme in the present invention includes proteases, esterases, carbohydrases etc. Specific examples of the proteases include pepsin, trypsin, chymotrypsin, collagenase, keratinase, elastase, subtilisin, papain, aminopeptidase and carboxypeptidase. Specific examples of the esterases include gastric lipase, pancreatic lipase, plant lipases, phospholipases, cholinesterases and phosphatases. The carbohydrases include cellulase, maltase, saccharase, amylase, pectinase and $\alpha$- and $\beta$-glycosidases. The isoelectric point of the enzyme is preferably 7 to 13, more preferably 8 to 12, even more preferably 9 to 11, from the viewpoint of a higher effect of the present invention. The enzyme in the present invention is preferably a protease, more preferably an alkali protease, from the viewpoint of higher usefulness of the enzyme and a higher effect of the present invention.

The enzyme concentration is defined in terms of the amount of a protein.

The microfiltration in the present invention is carried out using a microfiltration membrane. The type of the microfiltration membrane is not particularly limited insofar as the membrane is of such type as to filter a fluid with a sweeping stream flowing on the surface of the membrane, and the microfiltration membrane may be a flat membrane, a hollow fiber membrane, a tubular membrane etc. The membrane materials include, for example, ceramics such as alumina, titania, zirconia etc., inorganic membranes such as glass, metal etc., and organic membranes such as such as those based on cellulose acetate, nitrocellulose, aliphatic polyamide, polysulfone, polyolefin, polyacrylonitrile, polyether sulfone, polyvinyl chloride, polyvinyl alcohol, and fluorine polymer.

From the viewpoint of a sweeping stream on the surface of the separation membrane, the effect of the sweeping stream on the surface of the membrane is increased as the rate thereof on the surface of the membrane is increased. At a certain rate or more, however, pressure loss becomes significant, the consolidation of a gel layer occurs in the vicinity of the membrane, and the recovery of the enzyme in the membrane permeation flux and in the enzyme-containing solution is decreased. When the rate of the stream on the membrane surface is too low, pressure loss is decreased and the consolidation is avoided, but the effect thereof on release of a gel layer is reduced and the recovery of the enzyme in the enzyme-containing solution is lowered. From these viewpoints, the rate of the stream on the membrane surface is preferably 0.5 to 3 m/s, more preferably 0.5 to 1.5 m/s.

In the present invention, the transmembrane pressure difference in separation by microfiltration refers to average transmembrane pressure difference between an inlet and an outlet, and is usually preferably 0.2 MPa or less, more preferably 0.02 to 0.15 MPa. A transmembrane pressure difference outside of this range is not preferable because, when the transmembrane pressure difference is less than 0.02 MPa, the permeation flux tends to be decreased to deteriorate a throughput capacity. When the transmembrane pressure difference is more than 0.2 MPa, membrane clogging happens by consolidation of a gel layer on the membrane, etc., to lower the permeation flux. The method of giving transmembrane pressure difference may be carried out by applying a pressure on the side of a starting solution and/or reducing pressure on the side of a permeated solution. The operation temperature is usually 0 to 40°C, preferably 5 to 30°C. An operation temperature outside of this range is not preferable because when the operation temperature is less than 0°C, the viscosity of the enzyme-containing solution is increased thus sometimes decreasing the membrane permeation flux, and while when the operation temperature is more than 40°C, the properties of the enzyme-containing solution are deteriorated.

Not only a constant-pressure filtration system where the constant pressure as described above is applied while the permeation flux remains uncertain, but also a constant-flow filtration system where a filtration solution is pulled out at a constant flow rate is used in the operation method in the present invention. For keeping the permeability of the membrane high, an operation (backwashing) of periodically allowing a permeation solution to flow backward from the permeation side of the membrane may also be conducted.

In the present invention, a cationic surfactant should be added in an amount of 0.01 to 1% (w/v) to the enzyme-containing solution prior to microfiltration. Although the cationic surfactant used in the present invention is not particularly limited, a quaternary ammonium salt-type compound having a mono- or di-long-alkyl group is preferable from the viewpoint of working effect, and specific examples include compounds containing a long alkyl chain having 8 to 18 carbon atoms on the average, such as monoalkyl trimethyl ammonium chloride, monoalkyl trimethyl ammonium bromide, dialkyl dimethyl ammonium chloride, dialkyl dimethyl ammonium bromide, monoalkyl dimethyl benzyl ammonium chloride (benzalkonium chloride) etc. The average number of carbon atoms is preferably 10 to 18, more preferably 12 to 18, from the viewpoint of solubility in the enzyme-containing solution and the working effect.

The amount of the cationic surfactant added is defined by its net content. The amount of the cationic surfactant added to the enzyme-containing solution, in terms of the concentration thereof in the enzyme-containing solution, is preferably 0.01 to 1% (w/v), more preferably 0.02 to 0.5% (w/v), even more preferably 0.03 to 0.3% (w/v), from the viewpoint of improvement in enzyme permeability and improvement in permeation flux. The method of adding the cationic surfactant is not particularly limited, but from the viewpoint of its sufficient action on the enzyme-containing solution, the cationic surfactant is previously dissolved in a solvent such as water or isopropyl alcohol, and the solution is added, as it is, to the enzyme-containing solution and then stirred and mixed preferably by some means (for example, stirring with a stirring motor or liquid circulation with a pump) for about 10 minutes.

The amount of the cationic surfactant added in the present invention differs depending on the type and properties of the enzyme-containing solution. The amount of the cationic surfactant added, which is suitable depending on the type and properties of the enzyme-containing solution, is determined preferably by the following method. For determining the suitable amount, filtration may be conducted several times with the cationic surfactant added at varying concentrations. However, this method is troublesome, and thus the following easy filtration method may be used to determine a necessary amount of the cationic surfactant at one time. That is, filtration is carried out with entire circulation under predetermined conditions (permeation solution is returned as it is to the original solution so as not to change the activity of the original solution), and the permeation solution is filtered for about 2 hours until the filtration rate of the permeation solution, and the enzyme concentration, become constant. Once the filtration rate of the permeation solution and the enzyme concentration become constant, the permeation solution is sampled, and the concentration of the enzyme in the permeation solution, and the permeation flux, are measured. Immediately after sampling, the permeation solution is returned to the original solution, and the concentration of the enzyme in the original solution, and the amount of the solution, are kept as constant as possible. After the concentration and the amount become constant, the apparatus is once stopped. A predetermined amount of a cationic surfactant is added to the original solution. The mixture is mixed for 15 minutes. It is then similarly filtered again for 15 minutes. The concentration of the enzyme in the permeation solution, and the permeation flux, are measured. Thereafter, the cationic surfactant is added to the permeation solution to increase the concentration of the surfactant, to examine the relationship among the concentration of the surfactant, the permeability of the enzyme, and the permeation flux. By the operation described above, a suitable amount can be easily determined.

In the present invention, it is preferable that the cells are separated from a fermentation culture with a microbial cell density of more than 1 % (v/v), the resulting enzyme-containing solution is purified and concentrated by ultrafiltration, a cationic surfactant is added in an amount of 0.01 to 1% (w/v) to the resulting enzyme-containing solution having a cell density of 1% (v/v) or less and an enzyme concentration of 1% (w/v) or more in terms of the amount of proteins and then the mixture is subjected to ultrafiltration.

The fermentation culture in the present invention refers to a liquid containing fermentation products, that is, products accumulated as metabolites which microorganisms have produced by decomposing organic materials. The fermentation liquor may be for example cultures of microorganisms such as a bacterium, an actinomycete, a mold, a yeast etc., or may be cultures of animal or plant cells. Specific examples include products by alcohol fermentation, food fermentation for soy sauce, vinegar etc., antibiotic and anticancer substance fermentation, organic acid fermentation, amino acid fermentation, enzyme fermentation, etc. The fermentation culture is a culture having a cell density of higher than 1% (v/v), particularly 3% (v/v) or more, further 10 to 20% (v/v) The concentration of the enzyme in the fermentation culture is about 0.1 to 2% (w/v). In the fermentation culture at the end of main fermentation, cells of microorganisms occur at high density, so preferably the cells are first separated. In the present invention, the method of separating cells from a fermentation culture includes, for example, microfiltration, centrifugation, filter pressing, etc., and these methods can be combined, but a microfiltration membrane is preferably used.

After separation of cells from a fermentation culture, that is, after recovery of a fermentation product, the fermentation product is preferably concentrated by ultrafiltration. Ultrafiltration refers to a process of using an ultrafiltration membrane having a membrane pore, for example, in the range of 0.1 nm to 2 nm or a fractionation molecular weight in the range of 500 to 100,000 to prevent larger particles than that and high-molecular materials and make low-molecular substances pass through. By this operation, the enzyme in the fermentation product can be concentrated from 0.1 to 2 % (w/v) to 1 to 20% (w/v) or so.

The type and material of the ultrafiltration membrane used in the present invention are the same as described above in the microfiltration membrane. When the rate for sweeping on the surface of the ultrafiltration membrane for concentrating a fermentation liquor is increased, the sweeping effect is increased, but preferably the rate is 0.5 to 3 m/s. As the transmembrane pressure difference is increased, the filtration rate is increased, but when the transmembrane pressure difference is too high, a gel layer is consolidated in the vicinity of the membrane, thus preventing improvement in filtration rate. Accordingly, the transmembrane pressure difference is preferably 0.05 to 0.3 MPa. The operation temperature is 0 to 40°C, preferably 5 to 30°C. An operation temperature outside of this range is not preferable because when the operation temperature is less than 0°C, the viscosity of the fermentation product-containing solution is increased to deteriorate operativity, while at a temperature of higher than 40°C, the activity of the fermentation product is decreased.

In the operation of ultrafiltration, a purification with water added may be carried out by adding water, after the primary concentration, to improve the degree of purification and carry out a further concentration. When the salt concentration of the fermentation solution is decreased upon addition of water, the solubility of the enzyme may be decreased. In this case, an aqueous solution of calcium chloride or an aqueous solution of sodium sulfate may be used in place of water in purification with water added. Alternatively, these salts may be added to the concentrated solution after ultrafiltration.

Brief description of the drawing

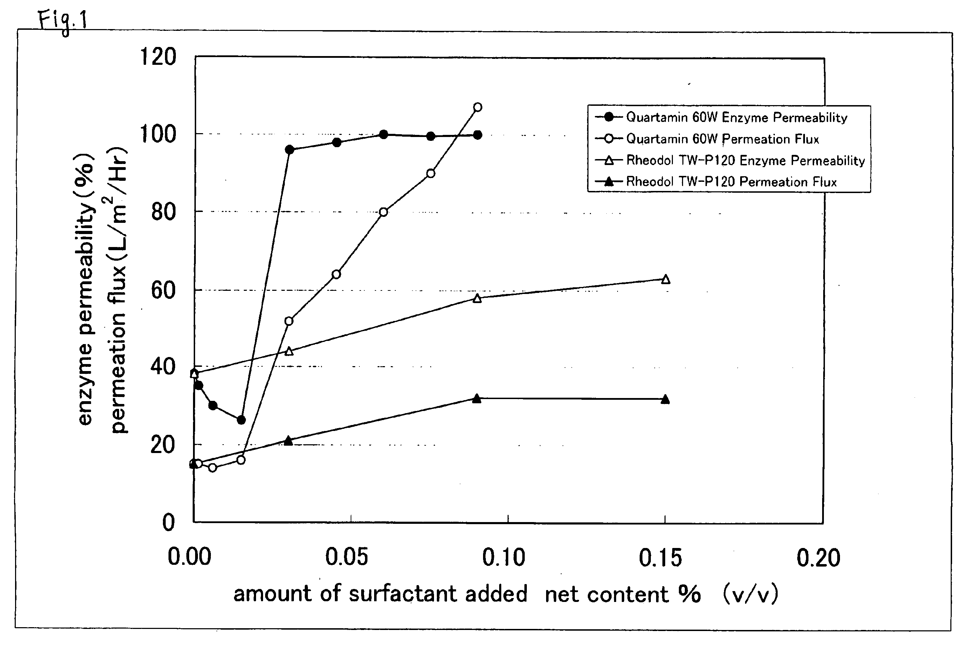

Fig. 1 is a graph showing the relationship among surfactant concentration, enzyme permeability, and membrane permeation flux.

Examples

The present invention is described in more detail by reference to Examples. The Examples are provided for illustrative purposes only and not intended to limit the present invention.

Examples 1 to 7, and Comparative Examples 1 and 2

100 L fermentation liquor (cell density 10.8% (v/v)) of an alkali protease (isoelectric point in the vicinity of 9.5) of *Bacillus* sp. KSM-9865 (FEM-P18566) was subjected to microfiltration (MF) at 10°C with a hollow fiber microfiltration membrane having an effective membrane area of 0.41 m$^2$, a pore diameter of 0.1 $\mu$m and a fiber inner diameter of 1. 9 mm$\phi$ (PSP-113L manufactured by Asahi Kasei Corporation). An operation of concentrating the fermentation liquor 3-fold, then adding an equal quantity of deionized water to the resulting concentrate and concentrating the resulting dilution again was repeated 3 times, to give an MF permeation solution in a volume of 168 L in total. The resulting MF permeation solution was subjected to ultrafiltration concentration (UF) with an ultrafiltration membrane with a fractionation molecular weight of 6000 (AIP-2013, with a membrane area of 1 m$^2$, manufactured by Asahi Kasei Corporation). An operation of concentrating the permeation solution 7-fold, then adding an equal quantity of 0.4% (v/v) aqueous calcium chloride to the resulting concentrate and concentrating the resulting dilution again was repeated 9 times, to give 23.5 L of an UF concentrate with a protein concentration of 4.8% (w/v). The density of microbial cells in the resulting UF concentrate was 0 % (v/v). Thereafter, sodium sulfate was dissolved at a final concentration of 2% (v/v) to the UF concentrate.

1.5 L of the above liquid was subjected to microfiltration at 10°C with a hollow fiber microfiltration membrane with an effective membrane area of 0.015 m$^2$, a pore diameter of 0.25 $\mu$m and a fiber inner diameter of 0.7 mm$\phi$ and a module length of 130 mm with 100 fibers (PSP-003 manufactured by Asahi Kasei Corporation). The treatment was carried out at a circulating flow rate of 1.8 L/min. (intramembrane linear velocity: 0.8 m/s) at an average pressure of 0.06 MPa, and then at intervals of 15 minutes, the membrane was backwashed from the permeation side of the membrane, and the average enzyme permeability and permeation flux, for 15 minutes from backwashing to next backwashing, were measured, and filtration was continued for about 2 hours until the enzyme permeability and permeation flux became constant. The permeation solution just after sampling was returned to an original-solution tank so that the enzyme concentration of the original solution and the amount of the solution were kept as constant as possible (hereinafter, this operation is referred to as "entire circulation operation"). After the enzyme permeability became constant, the apparatus was once stopped, and a surfactant shown in Table 1 was added in an amount shown in Table 1 to, and mixed for 15 minutes with, the permeation solution, and after the membrane was backwashed for 15 minutes again in the same manner, the enzyme permeability in the permeation solution and the permeation flux were measured. Thereafter, the cationic surfactant was similarly added to the permeation solution to increase the concentration of the surfactant, to examine the relationship among the concentration of the surfactant, the permeability of the enzyme, and the permeation flux. The permeability of the enzyme was defined by the following equation (1):

```
Enzyme permeability (%) =

[permeation solution activity (unit)/original solution

activity (unit)]×100        (1)
```

(Enzyme Activity Measurement Method - Casein Method)

1.0 ml of 50 mM boric acid buffer, pH 10, containing 1% (v/v) casein (hanmerstein, Merck) was kept at 30°C for 5 minutes, and then 0.1 ml enzyme solution was added thereto and reacted for 15 minutes. 2.0 ml of a reaction termination solution (0.11 M trichloroacetic acid-0.22 M sodium acetate-0.33 M acetic acid) was added thereto, left at room temperature for 10 minutes and filtered (filter paper No. 1, Advantec Toyo). An acid-soluble protein in the filtrate was quantified in the following manner by the method of Lowry et al. (J. Biol. Chem., 193, 265-275, 1981). 2.5 ml of an alkaline copper solution (1% potassium sodium tartrate : 1% copper sulfate : 1% sodium carbonate = 1 : 1 : 100) was added to 0.5 ml of the filtrate, and the mixture was left for 30°C for 10 minutes. 0.25 ml diluted phenol (2-fold dilution of a phenol reagent (Kanto Kagaku) with deionized water) was added and the resultant was left for 30 minutes at a constant temperature. An absorbance at 660 nm thereof was determined. 1 unit of the enzyme was regarded as the amount of the enzyme necessary for releasing an acid-soluble protein decomposition product corresponding to 1 mmol tyrosine for 1 minute in the reaction described above.

(Method of Measuring Protein Concentration)

By the method of Lowry *et al.*, the concentration of the protein was measured using bovine serum albumin as standard.

Table 1

| Surfactant | Trade name | Example No. | Surfactant concentration (pure content % (v/v)) | Enzyme permeability (%) | Permeation Flux (L/m$^2$/Hr) |
|---|---|---|---|---|---|
| ----- | ----- | Comparative example 1 | 0 | 44 | 40 |
| n-Octyl trimethyl ammonium chloride | Reagent (Tokyo Kasei Kogyo Co.,Ltd) | Example 1-1 | 0.015 | 74 | 59 |
| | | Example 1-2 | 0.03 | 76 | 56 |
| | | Example 1-3 | 0.06 | 84 | 53 |
| | | Example 1-4 | 0.09 | 71 | 45 |
| n-Decyl trimethyl ammonium chloride | Reagent (Tokyo Kasei Kogyo Co.,Ltd) | Example 2-1 | 0.015 | 100 | 85 |
| | | Example 2-2 | 0.03 | 100 | 91 |
| | | Example 2-3 | 0.06 | 89 | 80 |
| Lauryl trimethyl ammonium chloride | Quartamin 24P (Kao Corporation) | Example 3-1 | 0.015 | 90 | 99 |
| | | Example 3-2 | 0.03 | 100 | 72 |
| | | Example 3-3 | 0.06 | 97 | 61 |
| | | Example 3-4 | 0.09 | 100 | 125 |
| Cetyl trimethyl ammonium chloride | Quartamin 60W (Kao Corporation) | Example 4-1 | 0.03 | 100 | 123 |
| | | Example 4-2 | 0.06 | 100 | 227 |

(continued)

| Surfactant | Trade name | Example No. | Surfactant concentration (pure content % (v/v)) | Enzyme permeability (%) | Permeation Flux (L/m$^2$/Hr) |
|---|---|---|---|---|---|
| ----- | ----- | Comparative example 1 | 0 | 44 | 40 |
| Stearyl trimethyl ammonium chloride | Quartamin 86W (Kao Corporation) | Example 5-1 | 0.015 | 97 | 52 |
| | | Example 5-2 | 0.03 | 99 | 123 |
| | | Example 5-3 | 0.06 | 100 | 122 |
| Dialkyl(C8-18) dimethyl ammonium chloride | Sanisol C (Kao Corporation) | Example 6-1 | 0.03 | 66 | 37 |
| | | Example 6-2 | 0.06 | 98 | 75 |
| | | Example 6-3 | 0.09 | 100 | 373 |
| Dialkyl (C12-18) dimethyl ammonium chloride | Quartamin D2345P (Kao Corporation) | Example 7-1 | 0.03 | 57 | 36 |
| | | Example 7-2 | 0.06 | 56 | 37 |
| | | Example 7-3 | 0.09 | 87 | 43 |
| Sodium dodecyl sulfate | Emal O (Kao Corporation) | Comparative example 2-1 | 0.03 | 64 | 37 |
| | | Comparative example 2-2 | 0.06 | 69 | 20 |
| | | Comparative example 2-3 | 0.09 | 74 | 24 |

From the results in Table 1, it was found that the permeability of the enzyme and the permeation flux are significantly improved in microfiltration by adding a cationic surfactant. It was found that the anionic surfactant has a lower effect on improvement of enzyme permeability than the cationic surfactant and does not have an effect on improvement of permeation flux.

Example 8 and Comparative Examples 3 and 4

23.5 L of a solution obtained by dissolving sodium sulfate at a concentration of 2% in the alkali-protease UF concentrate (protein concentration 4.8% (w/v)) used in Example 1 was subjected to microfiltration at 10°C in the entire circulation operation with a hollow fiber microfiltration membrane with an effective membrane area of 0.2 m$^2$, a pore diameter of 0.25 $\mu$m and a fiber inner diameter of 0.7 mm$\phi$ and a module length of 347 mm with 400 fibers (PMP-102 manufactured by Asahi Kasei Corporation). The treatment was carried out at a circulating flow rate of 7.2 L/min. (intramembrane linear velocity: 0.8 m/s) at an average pressure of 0.05 MPa, and the same operation as in Example 1 was carried out. After the enzyme concentration became constant, the apparatus was once stopped, and a surfactant shown in Table 2 was added, in an amount shown in Table 2, to the original solution, mixed for 15 minutes, and after the membrane was backwashed for 15 minutes again in an analogous manner, the concentration of the enzyme in the permeation solution and the permeation flux were measured. A difference in the effect of each surfactant by varying concentration is shown in Fig. 1.

Table 2

| Surfactant | Trade name | Example No. | Surfactant concentration (pure content % (v/v)) | Enzyme Permeability (%) | Permeation Flux (L/m$^2$/Hr) |
|---|---|---|---|---|---|
| ----- | ----- | Comparative example 3 | 0 | 38.1 | 15 |
| Cetyl trimethyl ammonium chloride | Quartamin 60W (Kao Corporation) | Example 8-1 | 0.0015 | 34.9 | 15 |
| | | Example 8-2 | 0.006 | 29.9 | 14 |
| | | Example 8-3 | 0.015 | 26.2 | 16 |
| | | Example 8-4 | 0.030 | 96.0 | 52 |
| | | Example 8-5 | 0.045 | 97.9 | 64 |
| | | Example 8-6 | 0.060 | 100.0 | 80 |
| | | Example 8-7 | 0.075 | 99.6 | 90 |
| | | Example 8-8 | 0.090 | 100.0 | 107 |
| Polyoxyethylene sorbitan monopalmitate | Rheodol TW-P120 (Kao Corporation) | Comparative example 4-1 | 0.03 | 44.1 | 21 |
| | | Comparative example 4-2 | 0.09 | 58.1 | 32 |
| | | Comparative example 4-3 | 0.15 | 63.2 | 32 |

From the results in Table 2 and Fig. 1, it was found that even if the scale of the membrane is changed, the permeability of the enzyme and the permeation flux are significantly improved in microfiltration by similarly adding a cationic surfactant. The nonionic surfactant has a lower effect on improvement of enzyme permeability than the cationic surfactant and has a lower effect on improvement of permeation flux.

Example 9 and Comparative Example 5

The same operation as in Comparative Example 1 and Example 4-1 was carried out except that an UF concentrate (cell density 0% (v/v), protein concentration 5.6% (w/v)) of an alkali protease (isoelectric point in the vicinity of 10.2) of *Bacillus* sp. KSM-K16 in place of the alkali protease (isoelectric point in the vicinity of 9.5) of *Bacillus* sp. KSM-9865 (FEM-P18566) was used. The results are shown in Table 3.

Table 3

| Surfactant | Trade name | Example No. | Surfactant concentration (Pure content % (v/v)) | Enzyme Permeability (%) | Permeation Flux (L/m$^2$/Hr) |
|---|---|---|---|---|---|
| ----- | ----- | Comparative example 5 | 0 | 5 | 35 |
| Cetyl trimethyl ammonium chloride | Quartamin 60W (Kao Corporation) | Example 9 | 0.03 | 100 | 112 |

From the results in Table 3, it was found that even if the enzyme is changed, the permeability of the enzyme and the permeation flux are significantly improved in microfiltration by adding a cationic surfactant.

**Claims**

1. A process for producing an enzyme, comprising the steps of recovering an enzyme by adding a cationic surfactant in an amount of 0.01 to 1% (w/v) to an enzyme-containing solution, having a cell density of 1% (v/v) or less and an enzyme concentration of 1% (w/v) or more in terms of the amount of proteins, and microfiltering the enzyme-containing solution.

2. A process for producing an enzyme, comprising the steps of separating microbial cells from a fermentation culture having a cell density of more than 1% (v/v), purifying and concentrating the resulting enzyme-containing solution by ultrafiltration, adding a cationic surfactant in an amount of 0.01 to 1% (w/v) to the resulting enzyme-containing solution having a cell density of 1% (v/v) or less and an enzyme concentration of 1% (w/v) or more in terms of the amount of proteins and microfiltering the mixture.

**Patentansprüche**

1. Verfahren zur Herstellung eines Enzyms, umfassend die Schritte der Rückgewinnung eines Enzyms durch Hinzufügen eines kationischen Tensids in einer Menge von 0,01 bis 1 % (G/V) zu einer enzymhaltigen Lösung mit einer Zelldichte von 1 % (V/V) oder weniger und einer Enzymkonzentration im Sinne der Menge der Proteine von 1 % (G/V) oder mehr, und Mikrofiltrieren der enzymhaltigen Lösung.

2. Verfahren zur Herstellung eines Enzyms, umfassend die Schritte der Abtrennung von mikrobiellen Zellen von einer Fermentationskultur mit einer Zelldichte von mehr als 1 % (V/V); Reinigung und Konzentrieren der resultierenden enzymhaltigen Lösung durch Ultrafiltration, Hinzufügen eines kationischen Tensids in einer Menge von 0,01 bis 1 % bis (G/V) zu der enzymhaltigen Lösung mit einer Zelldichte von 1 % (V/V) oder weniger und einer Enzymkonzentration im Sinne der Menge der Proteine von 1 % (G/V) oder mehr, und Mikrofiltrieren der Mischung.

**Revendications**

1. Processus pour produire une enzyme, comprenant les étapes de récupération d'une enzyme en ajoutant un agent de surface cationique en une quantité de 0,01 à 1 % (p/v) à une solution contenant une enzyme, ayant une densité cellulaire de 1 % (v/v) ou moins et une concentration d'enzyme de 1 % (p/v) ou plus en termes de la quantité de protéines, et de microfiltrage de la solution contenant une enzyme.

2. Processus pour produire une enzyme, comprenant les étapes de séparation de cellules microbiennes à partir d'une culture de fermentation ayant une densité cellulaire de plus de 1 % (v/v), d'épuration et de concentration de la solution résultante contenant une enzyme par ultrafiltration, d'ajout d'un agent de surface cationique en une quantité de 0,01 à 1 % (p/v) à la solution résultante contenant une enzyme ayant une densité cellulaire de 1 % (v/v) ou moins et une concentration d'enzyme de 1 % (p/v) ou plus en termes de la quantité de protéines et de microfiltrage du mélange.

Fig.1

EP 1 930 419 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11318482 A
- JP 4354585 A
- JP 2004089155 A

**Non-patent literature cited in the description**

- **Lowry et al.** *J. Biol. Chem.,* 1981, vol. 193, 265-275